# EUROPÄISCHE PATENTSCHRIFT

(11) **EP 1 873 521 B1**
(45) Veröffentlichungstag und Bekanntmachung des Hinweises auf die Patenterteilung: **21.09.2011**
(21) Anmeldenummer: 06013199.2
(22) Anmeldetag: 27.06.2006
(51) Int. Cl.: G01N 33/487, B29C 45/14

(54) **Diagnostische Bandkassette**
Diagnostic test band cassette
Cassette à bande diagnostique

(43) Veröffentlichungstag der Anmeldung: 02.01.2008
(73) Patentinhaber: F.Hoffmann-La Roche AG, 4070 Basel (CH); Roche Diagnostics GmbH, 68305 Mannheim (DE)
(72) Erfinder: Sacherer, Klaus Dieter, 67281 Kirchheim (DE)
(74) Vertreter: Pfiz, Thomas

(56) Entgegenhaltungen:
- WO-A-2004/056269
- DE-A1- 4 339 450
- DE-A1-102004 024 970
- US-A- 4 218 421
- HAACK U: "OUTSERT-TECHNIK, VERFAHREN ZUR WIRTSCHAFTLICHEN HERSTELLUNG FEINWERKTECHNISCHER BAUTEILE" FEINWERKTECHNIK + MESSTECHNIK, HANSER, MUNCHEN., DE, Bd. 87, Nr. 6, September 1979 (1979-09), Seiten 253-259, XP001173671 ISSN: 0340-1952

## Beschreibung

Die Erfindung betrifft eine diagnostische Bandkassette insbesondere für Blutzuckertests mit einem Testband, welches mit einer Vielzahl von Testfeldern zur Untersuchung von Körperflüssigkeit versehen ist, und einem Gehäuse zur Aufnahme des Testbands. Gegenstand der Erfindung ist auch ein Herstellungsverfahren für eine solche Bandkassette.

Für die Selbstdiagnose von Diabetikern werden bislang in der Praxis einzelne Teststreifen eingesetzt, die nach der Applikation einer geringen Probenmenge photometrisch oder elektrochemisch untersucht werden, um den Glucosegehalt in der Probe (Blut bzw. Gewebeflüssigkeit) möglichst genau und zuverlässig zu bestimmen. Zur Verbesserung der Anwenderfreundlichkeit wurde bereits vorgeschlagen, eine Vielzahl von Tests auf einem Testband in Form einer Bandkassette bereitzustellen. Solche Bandkassetten sollen sich als Einwegteil in kompakte Handgeräte einsetzen lassen, um alle erforderlichen Untersuchungsschritte automatisch und schnell ausführen zu können. Hierbei ist zu beachten, dass die Verbrauchsteile ein Massenprodukt darstellen, an welches aufgrund der erforderlichen Zuverlässigkeit hohe Anforderungen gestellt werden.

Ausgehend hiervon liegt der Erfindung die Aufgabe zugrunde, die im Stand der Technik vorgeschlagenen Bandkassetten für den disposiblen Einsatz weiter zu verbessern und bei einfacher Herstellbarkeit eine hohe Lager- und Gebrauchsstabilität zu erreichen.

Zur Lösung dieser Aufgabe wird die im Patentanspruch 1 bzw. 18 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Funktionalität der Bandkassette mit möglichst wenig separaten Bauteilen zu gewährleisten. Dementsprechend wird gemäß der Erfindung vorgeschlagen, dass das Gehäuse ein in Outsert-Technik aus einem Metallträger und angespritztem Kunststoff gebildetes Outsert-Moulding-Teil aufweist. Die Outsert-(Moulding)-Technik erlaubt die kostengünstige Herstellung von Metall-Kunststoff-Verbundteilen, wobei Materialbrücken und Hinterschneidungen eine feste Verbindung gewährleisten. Im Hinblick auf eine diagnostische Bandkassette können so gehäuseintegrierte Funktionselemente zu besonderen Zwecken auch in dem Metallträger geschaffen werden, ohne dass gesonderte Bauteile gefertigt und manipuliert werden müssten. Speziell werden durch den Metallträger Formveränderungen, wie sie bei bloßen Plastikteilen auftreten, auch bei lang dauernder Vorratshaltung bzw. Geräteeinsatzzeit vermieden, wobei auch mit geringen Wandstärken eine hohe Gehäusefestigkeit erzielt wird. Damit lassen sich kompakte Bauformen bei hoher Magazinierungsdichte der Tests produktionstechnisch einfach realisieren.

Die in das Outsert-Moulding-Teil integrierten Funktionselemente umfassen vorzugsweise Feder-, Verbindungs- und/oder Führungselemente für eine besonders zweckmäßige Kassettengestaltung.

Besondere Vorteile hinsichtlich einer Beeinflussung von Bandlauffunktionen bieten sich dadurch, dass die Funktionselemente mindestens ein an dem Metallträger ausgebildetes Federelement umfassen. Dieses muss dann nicht mehr gesondert in das Gehäuse eingesetzt werden, sondern kann vielmehr in einem Arbeitsgang in der gewünschten Stellung in das Outsert-Moulding-Teil integriert werden.

Eine weitere vorteilhafte Ausführung sieht vor, dass der Metallträger aus einem Blechzuschnitt vorgeformt ist, so dass sich dünne und dennoch tragfähige Gehäusewandungen verwirklichen lassen.

Vorteilhafterweise ist ein Zuschnittteil des Blechzuschnitts als elastisch rückstellbares Federelement gebogen. Dabei ist es von besonderem Vorteil, wenn ein Funktionselement in Form einer Blattfeder für eine Dichtfunktion gegenüber dem Testband vorgesehen ist.

Eine weitere Vereinfachung sieht vor, dass die Blattfeder an einem freien Ende durch eine Wandung aus angespritztem Kunststoff in einer vorgespannten Stellung lösbar gehalten ist und ggf. erst im Zuge der Endmontage in die vorgesehene Funktionsstellung gebracht wird.

Bevorzugt ist es auch, wenn ein Funktionselement in Form einer Spiralfeder für eine Federbeaufschlagung einer Bandspule vorhanden ist. Hierbei ist es günstig, wenn die Spiralfeder gegenüber einer Ebene des Metallträgers vertieft und wendelförmig in das Gehäuseinnere ausgebogen ist. Ein weiterer Vorteil auch zum Schutz ges Gehäuseinnenraums vor Verschmutzung wird dadurch erreicht, dass die Spiralfeder durch eine Abdeckung aus angespritztem Kunststoff gegen einen Eingriff von außen abgedeckt ist.

Um die Federfunktion und Positionierung manipulieren zu können, ist es vorteilhaft, wenn der angespritzte Kunststoff im Bereich des Federelements mindestens einen Wanddurchbruch für einen auf das Federelement einwirkenden Ausdrücker und/oder Zentrierstift aufweist.

Eine weitere vorteilhafte Ausgestaltung sieht vor, dass die Funktionselemente mindestens ein Verankerungselement für das Outsert-Moulding-Teil umfassen. Dies lässt sich besonders einfach dadurch realisieren, dass an dem Metallträger abstehende Stützhaken mit Kunststoff in Form eines Rastelements umspritzt sind.

Eine weitere vorteilhafte Gehäusefunktion wird dadurch erreicht, dass die Funktionselemente eine Abdeckung für das zwischen zwei Bandspulen transportierte Testband umfassen. Vorteilhafterweise kann die Abdeckung durch mit Kunststoff umspritzte, vorzugsweise hinterschnittene Laschen des Metallträgers gebildet sein.

Um den Bandlauf mit möglichst geringen Reibungsverlusten zu gewährleisten, ist es von Vorteil, wenn die Funktionselemente eine Bandumlenkung zur Gleitführung des Testbands umfassen.

Vorteilhafterweise bildet das Outsert-Moulding-Teil ein Gehäuseteil, insbesondere einen Kassettendeckel oder einen Kassettenkörper für die Bandkassette.

In verfahrensmäßiger Hinsicht wird die eingangs genannte Aufgabe dadurch gelöst, dass ein Metallträger vorzugsweise als Blechzuschnitt vorgeformt wird und durch Spritzgießen in Outsert-Technik mit angespritzten Kunststoffpartien versehen wird, wobei in das so gebildete Outsert-Moulding-Teil Funktionselemente für die Bandkassette integriert werden, und wobei durch das Outsert-Moulding-Teil ein Teil eines Gehäuses gebildet wird, in welchem ein Testband aufgenommen wird.

Im Folgenden wird die Erfindung anhand eines in der Zeichnung schematisch dargestellten Ausführungsbeispiels näher erläutert. Es zeigen
- Fig. 1: eine Bandkassette für Blutzuckertests in einer perspektivischen Darstellung;
- Fig. 2: einen Metallträger für ein Deckelteil der Bandkassette in perspektivischer Innenansicht; und
- Fig. 3: das in Outsert-Technik gebildete Deckelteil in Fig. 2 entsprechender Darstellung.

Die in der Zeichnung dargestellte Bandkassette 10 ermöglicht die Durchführung einer Mehrzahl von Glucoseuntersuchungen an vor Ort vom Patienten selbst gewonnenen Blutproben. Zu diesem Zweck ist ein Gehäuse 12 zur Aufnahme eines Testbands 14 ausgebildet, welches mit einer Vielzahl von Testfeldern 16 versehen ist, die sich sukzessive an einer Applikationsspitze 18 mit Blut beaufschlagen lassen. Der Bandtransport erfolgt dabei über zwei in das Gehäuse 12 eingesetzte Bandspulen 20, 22. Der prinzipielle Untersuchungsablauf ergibt sich beispielsweise aus der WO 2004/056269.

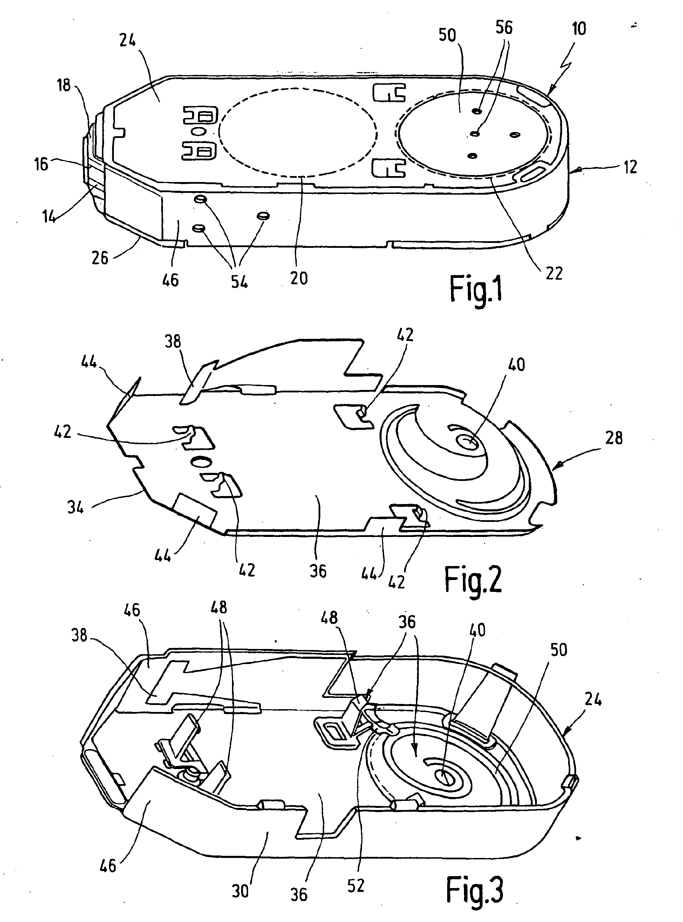

Das Gehäuse 12 umfasst ein als Deckelteil ausgebildetes Outsert-Moulding-Teil 24 und einen damit verbindbaren Kassettenkörper 26. Das Outsert-Moulding-Teil 24 ist in Outsert-Technik aus einem Metallträger 28 gemäß Fig. 2 und daran angespritztem Kunststoff 30 gemäß Fig. 3 gebildet. Auf diese Weise können an dem Fertigteil verschiedene Funktionsteile 32 produktions- und anwendungstechnisch vorteilhaft integriert werden. Bei der Outsert-Technik wird der Metallträger 28 in den Werkzeughohlraum eines schließbaren Formwerkzeugs eingelegt und vorzugsweise durch Spritzgießen mit aushärtbarer Kunststoffmasse umschlossen. Dabei wird der Kunststoff über Hinterschneidungen und Durchbrüche an dem Metallträger fest verankert. Das Verfahren als solches ist dem Fachmann bekannt, so dass weitere Verfahrensdetails hier nicht beschrieben werden müssen.

Fig. 2 zeigt den als Metallblechzuschnitt 34 durch Stanzen und Biegen geformten Metallträger 28. Das Zuschnittteil 34 besitzt eine ebene Grundfläche 36, aus welcher verschiedene funktionelle Teile, insbesondere Federelemente 38, 40 und Stützelemente 42, 44 herausgebogen sind.

An einer abgekanteten Seitenfläche ist eine Blattfeder 38 für eine verbesserte Dichtfunktion gegenüber dem über eine nicht gezeigte Dichtung vorbeigeführten Testband 14 vorgesehen. Zur Beaufschlagung des Bandwickels 22 mit einer Federkraft ist im Bereich der Grundfläche 36 eine Spiralfeder 40 angeordnet, wobei das angeformte Federende kreisförmig tiefgezogen ist und das freie Federende wendelförmig zum Gehäuseinneren hin ausgebogen ist.

Für eine Stabilisierung von angespritzten Verankerungselementen sind mehrere abgewinkelte, seitliche hinterschnittene Stützhaken 42 an der Grundfläche 36 verteilt angeordnet. Randseitig an der Grundfläche 36 sind Laschen 44 als seitliche Stützelemente vorgesehen.

Das fertige Outsert-Moulding-Teil 24 weist gemäß Fig. 3 durch die Laschen 44 gestützte Seitenwandungen 46 auf, welche das Testband 14 mit Ausnahme des Bereichs der Applikationsspitze 18 nach außen abdecken. Zur Verrastung mit dem Kassettenkörper 26 sind Rasthaken 48 aus Kunststoff auf die Stützhaken 42 aufgespritzt. Die Spiralfeder 40 wird durch einen Deckel 50 aus Kunststoff in der Ebene der Grundfläche 36 gegen einen Eingriff von außen abgedeckt. Dieser bleibt durch einen gegenüberliegenden Anspritzpunkt 52 an der Innenseite der Grundfläche 36 mit dem Metallträger 28 fest verbunden.

Im Spritzgießwerkzeug wird die Blattfeder 38 in den Bereich der angrenzenden Seitenwand 46 vorgespannt und dort durch Zentrierstifte fixiert, bis der verfestigte Kunststoff aufgrund seines Schrumpfverhaltens die Haltefunktion übernimmt. Die Zentrierstifte bilden dabei Wanddurchbrüche 54 in dem Kunststoff, wie sie auf der Außenseite des Deckelteils 24 in Fig. 1 erkennbar sind. Bei der Endmontage kann dann die Feder 38 über einen durch die Durchbrüche 54 hindurchgreifenden Ausdrücker in die Wirkstellung nach innen ausgedrückt werden. Entsprechende Durchbrüche 56 sind auch im Bereich der Abdeckung 50 der Spiralfeder 40 vorhanden.

Grundsätzlich ist es auch möglich, dass weitere Teile der Kassette 10 in Outsert-Technik ausgebildet werden. Speziell kann der Kassettenkörper 26 einen Metallträger aufweisen, an welchem Bandumlenkungen für das Testband 14 ausgeformt sind. Diese können nach dem Anspritzen der aus Kunststoff gebildeten Körperteile außenseitig freigehalten werden, so dass das zwischen den Spulen 20, 22 über die Spitze 18 geführte Testband auf den metallischen Bandumlenkungen mit geringer Reibung gleitet.

## Patentansprüche

1. Diagnostische Bandkassette, insbesondere für Blutzuckertests, mit einem Testband (14), welches mit einer Vielzahl von Testfeldern (16) zur Untersuchung von Körperflüssigkeit versehen ist, und einem Gehäuse (12) zur Aufnahme des Testbands (14), **dadurch gekennzeichnet, dass** das Gehäuse (12) mindestens ein aus einem Metallträger (28) und angespritztem Kunststoff (30) gebildetes Outsert-Moulding-Teil (24) aufweist.

2. Bandkassette nach Anspruch 1, **dadurch gekennzeichnet, dass** das Outsert-Moulding-Teil (24) integrierte Funktionselemente (36) aufweist.

3. Bandkassette nach Anspruch 2, **dadurch gekennzeichnet, dass** die Funktionselemente (36) mindestens ein an dem Metallträger (28) ausgebildetes Federelement (38,40) umfassen.

4. Bandkassette nach einem der Ansprüche 1 bis 3, **dadurch gekennzeichnet, dass** der Metallträger (28) aus einem Blechzuschnitt (34) vorgeformt ist.

5. Bandkassette nach Anspruch 4, **dadurch gekennzeichnet, dass** ein Zuschnittteil des Blechzuschnitts (34) als elastisch rückstellbares Federelement (38,40) gebogen ist.

6. Bandkassette nach einem der Ansprüche 2 bis 5, **dadurch gekennzeichnet, dass** ein Funktionselement (36) in Form einer Blattfeder (38) für eine Dichtfunktion gegenüber dem Testband (14) vorgesehen ist.

7. Bandkassette nach Anspruch 6, **dadurch gekennzeichnet, dass** die Blattfeder (38) an einem freien Ende durch eine Wandung (46) aus angespritztem Kunststoff (30) in einer vorgespannten Stellung lösbar gehalten ist.

8. Bandkassette nach einem der Ansprüche 2 bis 7, **dadurch gekennzeichnet, dass** ein Funktionselement (36) in Form einer Spiralfeder (40) für eine Federbeaufschlagung einer Bandspule (22) vorgesehen ist.

9. Bandkassette nach Anspruch 8, **dadurch gekennzeichnet, dass** die Spiralfeder (40) gegenüber einer Ebene des Metallträgers (28) vertieft und wendelförmig in das Gehäuseinnere ausgebogen ist.

10. Bandkassette nach Anspruch 8 oder 9, **dadurch gekennzeichnet, dass** die Spiralfeder (40) durch einen Deckel (50) aus angespritztem Kunststoff (30) gegen einen Eingriff von außen abgedeckt ist.

11. Bandkassette nach einem der Ansprüche 3 bis 10, **dadurch gekennzeichnet, dass** der angespritzte Kunststoff (30) im Bereich des Federelements (38,40) mindestens einen Wanddurchbruch (54,56) für einen auf das Federelement (38,40) einwirkenden Ausdrücker und/oder Zentrierstift aufweist.

12. Bandkassette nach einem der Ansprüche 2 bis 11, **dadurch gekennzeichnet, dass** die Funktionselemente (36) mindestens ein Verankerungselement (48) für das Gehäuseteil (24) umfassen.

13. Bandkassette nach einem der Ansprüche 1 bis 12, **dadurch gekennzeichnet, dass** an dem Metallträger (28) abstehende Stützhaken (42) mit Kunststoff in Form eines Rastelements umspritzt sind.

14. Bandkassette nach einem der Ansprüche 2 bis 13, **dadurch gekennzeichnet, dass** die Funktionselemente (36) eine Abdeckung (46) für das zwischen zwei Bandspulen transportierte Testband (14) umfassen.

15. Bandkassette nach Anspruch 14, **dadurch gekennzeichnet, dass** die Abdeckung (46) durch mit Kunststoff (30) umspritzte, vorzugsweise hinterschnittene Laschen (44) des Metallträgers (28) gebildet ist.

16. Bandkassette nach einem der Ansprüche 2 bis 15, **dadurch gekennzeichnet, dass** die Funktionselemente (36) eine Bandumlenkung zur Gleitführung des Testbands (14) umfassen.

17. Bandkassette nach einem der Ansprüche 1 bis 16, **dadurch gekennzeichnet, dass** das Outsert-Moulding-Teil (24) ein Gehäuseteil, insbesondere einen Kassettendeckel (24) oder einen Kassettenkörper (26) bildet.

18. Verfahren zur Herstellung einer diagnostischen Bandkassette (10), insbesondere für Blutzuckertests, nach einem der vorhergehenden Ansprüche, bei welchem ein Metallträger (28), vorzugsweise als Blechzuschnitt (34), vorgeformt wird und durch Spritzgießen in Outsert-Technik mit angespritzten Kunststoffpartien (46,48,50) versehen wird, wobei in das so gebildete Outsert-Moulding-Teil (24) Funktionselemente (36) für die Bandkassette (10) integriert werden, und wobei durch das Outsert-Moulding-Teil (24) ein Teil eines Gehäuses (12) gebildet wird, in welchem ein Testband (14) aufgenommen wird.

## Claims

1. Diagnostic tape cassette, especially for blood sugar tests, comprising a test tape (14) which is provided with a plurality of test fields (16) for analysing body fluid and a housing (12) for receiving the test tape (14), **characterized in that** the housing (12) has at least one outsert moulding part (24) formed from a metal support (28) and moulded-on plastic (30).

2. Tape cassette according to claim 1, **characterized in that** the outsert moulding part (24) has integrated functional elements (36).

3. Tape cassette according to claim 2, **characterized in that** the functional elements (36) comprise at least one spring element (38, 40) formed on the metal support (28).

4. Tape cassette according to one of the claims 1 to 3, **characterized in that** the metal support (28) is preformed from a precut metal sheet blank (34).

5. Tape cassette according to claim 4, **characterized in that** a precut part of the metal sheet blank (34) is bent as an elastically resettable spring element (38, 40).

6. Tape cassette according to one of the claims 2 to 5, **characterized in that** a functional element (36) is provided in the form of a leaf spring (38) for a sealing function with respect to the test tape (14).

7. Tape cassette according to claim 6, **characterized in that** a free end of the leaf spring (38) is detachably held in a pretensioned position by a wall (46) made of moulded-on plastic (30).

8. Tape cassette according to one of the claims 2 to 7, **characterized in that** a functional element (36) in the form of a spiral spring (40) is provided to spring-load a tape reel (22).

9. Tape cassette according to claim 8, **characterized in that** the spiral spring (40) is recessed relative to a plane of the metal support (28) and is bent out into the interior of the housing in a coil shape.

10. Tape cassette according to claim 8 or 9, **characterized in that** the spiral spring (40) is covered by a cover (50) made of moulded-on plastic (30) against contact from outside.

11. Tape cassette according to one of the claims 3 to 10, **characterized in that** the moulded-on plastic (30) in the area of the spring element (38, 40) has at least one opening in the wall (54, 56) for a pusher and/or centring pin acting on the spring element (38, 40).

12. Tape cassette according to one of the claims 2 to 11, **characterized in that** the functional elements (36) comprise at least one anchoring element (48) for the housing part (24).

13. Tape cassette according to one of the claims 1 to 12, **characterized in that** supporting hooks (42) that protrude from the metal support (28) are coated with plastic in the form of a snap-in element.

14. Tape cassette according to one of the claims 2 to 13, **characterized in that** the functional elements (36) comprise a cover (46) for the test tape (14) transported between two tape reels.

15. Tape cassette according to claim 14, **characterized in that** the cover (46) is formed by preferably undercut flaps (44) of the metal support (28) that are coated with plastic (30).

16. Test tape according to one of the claims 2 to 15, **characterized in that** the functional elements (36) comprise a tape deflector as a guide slide bearing for the test tape (14).

17. Tape cassette according to one of the claims 1 to 16, **characterized in that** the outsert moulding part (24) forms a housing part and in particular a cassette cover (24) or a cassette body (26).

18. Process for producing a diagnostic tape cassette (10), in particular for blood sugar tests, according to one of the previous claims, in which a metal support (28) is preformed preferably as a metal sheet blank (34) and provided with moulded-on plastic parts (46, 48, 50) using the outsert technique wherein functional elements (36) for the tape cassette (10) are integrated into the outsert moulding part (24) that is formed in this manner, and wherein by means of the outsert moulding part (24) a housing part is formed in which a test tape (14) is received.

## Revendications

1. Cassette à bande diagnostique, en particulier pour tests de glycémie, avec une bande de test (14) pourvue d'une pluralité de zones de test (16) pour analyser un liquide corporel, et avec un boîtier (12) pour recevoir la bande de test (14), **caractérisée en ce que** le boîtier (12) présente au moins une pièce moulée en technique outsert (24) formée d'un support métallique (28) et de matière plastique surmoulée (30) .

2. Cassette à bande selon la revendication 1, **caractérisée en ce que** la pièce moulée en technique outsert (24) présente des éléments fonctionnels intégrés (36).

3. Cassette à bande selon la revendication 2, **caractérisée en ce que** les éléments fonctionnels (36) comprennent au moins un élément ressort (38, 40) formé sur le support métallique (28).

4. Cassette à bande selon l'une des revendications 1 à 3, **caractérisée en ce que** le support métallique (28) est préformé à partir d'une pièce découpée de tôle (34).

5. Cassette à bande selon la revendication 4, **caractérisée en ce qu'**une partie de pièce découpée de la pièce découpée de tôle (34) est pliée sous forme d'élément ressort (38, 40) à rappel élastique.

6. Cassette à bande selon l'une des revendications 2 à 5, **caractérisée en ce qu'**un élément fonctionnel (36) sous forme de ressort à lame (38) est prévu pour une fonction d'étanchéité par rapport à la bande de test (14).

7. Cassette à bande selon la revendication 6, **caractérisée en ce que** le ressort à lame (38), à une extrémité libre, est maintenu de manière amovible dans une position précontrainte par une paroi (46) en matière plastique surmoulée (30).

8. Cassette à bande selon l'une des revendications 2 à 7, **caractérisée en ce qu'**un élément fonctionnel (36) sous forme de ressort en spirale (40) est prévu pour une sollicitation par ressort d'une bobine de bande (22).

9. Cassette à bande selon la revendication 8, **caractérisée en ce que** le ressort en spirale (40) est en retrait par rapport à un plan du support métallique (28) et plié de manière hélicoïdale vers l'intérieur du boîtier.

10. Cassette à bande selon la revendication 8 ou 9, **caractérisée en ce que** le ressort en spirale (40) est protégé contre une intervention de l'extérieur par un couvercle (50) en matière plastique surmoulée (30).

11. Cassette à bande selon l'une des revendications 3 à 10, **caractérisée en ce que** la matière plastique surmoulée (30) présente au niveau de l'élément ressort (38, 40) au moins une ouverture de paroi (54, 56) pour un éjecteur et/ou une goupille de centrage agissant sur l'élément ressort (38, 40).

12. Cassette à bande selon l'une des revendications 2 à 11, **caractérisée en ce que** les éléments fonctionnels (36) comprennent au moins un élément d'ancrage (48) pour la partie de boîtier (24).

13. Cassette à bande selon l'une des revendications 1 à 12, **caractérisée en ce que** des crochets de support (42) en saillie sur le support métallique (28) sont enrobés de matière plastique sous forme d'élément d'encliquetage.

14. Cassette à bande selon l'une des revendications 2 à 13, **caractérisée en ce que** les éléments fonctionnels (36) comprennent un recouvrement (46) pour la bande de test (14) transportée entre deux bobines de bande.

15. Cassette à bande selon la revendication 14, **caractérisée en ce que** le recouvrement (46) est formé par des languettes (44) enrobées de matière plastique (30), de préférence en contre-dépouille, du support métallique (28).

16. Cassette à bande selon l'une des revendications 2 à 15, **caractérisée en ce que** les éléments fonctionnels (36) comprennent un renvoi de bande pour le guidage coulissant de la bande de test (14).

17. Cassette à bande selon l'une des revendications 1 à 16, **caractérisée en ce que** la pièce moulée en technique outsert (24) forme une partie de boîtier, en particulier un couvercle de cassette (24) ou un corps de cassette (26).

18. Procédé de fabrication d'une cassette à bande diagnostique (10), en particulier pour tests de glycémie, selon l'une des revendications précédentes, dans lequel un support métallique (28), de préférence sous forme de pièce découpée de tôle (34), est préformé et muni de parties plastiques surmoulées (46, 48, 50) par moulage par injection en technique outsert, des éléments fonctionnels (36) pour la cassette à bande (10) étant intégrés dans la pièce moulée en technique outsert (24) ainsi formée et ladite pièce moulée en technique outsert (24) formant une partie d'un boîtier (12) dans laquelle est reçue une bande de test (14).
